# EUROPEAN PATENT APPLICATION

(11) **EP 2 905 286 A1**
(43) Date of publication of application: **12.08.2015**
(21) Application number: 14154400.7
(22) Date of filing: 08.02.2014
(51) Int. Cl.: C07F 15/00, A61P 35/00, A61K 31/282

(54) **Novel diazabicyclohydrocarbon-Pt complexes and the use thereof as pharmaceuticals**

(71) Applicant: Studiengesellschaft Kohle mbH, 45470 Mülheim (DE)
(72) Inventor: Pörschke, Klaus-Richard, 45478 Mülheim (DE); Cui, Huiling, 45478 Mülheim (DE)

(57) **Abstract**

The present invention relates to platinum complexes with diazabicyclohydrocarbon compounds of the general formula (I), processes for preparing said complexes and the use thereof as pharmaceutically active compounds, in particular as cytostatic compounds.

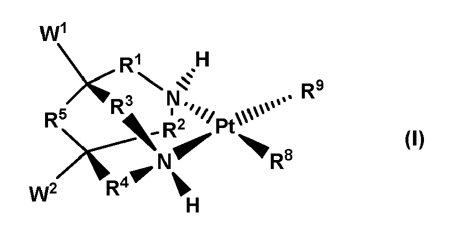

## Description

The present invention relates to platinum complexes with diazabicyclohydrocarbon compounds, processes for preparing said complexes and the use thereof as pharmaceutically active compounds, in particular as cytostatic compounds.

The discovery of the antitumor activity of cisplatin, cis-(NH₃)₂PtCl₂, prompted an intense screening of related Pt complexes in a search of possibly more effective and at the same time less toxic analogues. In chemotherapy, the prototypical cisplatin is particularly active in the treatment of testicular and ovarian cancers and also in the treatment of bladder, head, and neck cancers, as well as small cell and non-small cell lung cancer. Substantial side-effects are dose-limiting nephrotoxicity, gastrointestinal toxicity, ototoxicity, neurotoxicity, and myelosuppression. Patients experience inter alia nausea, vomiting, and anorexia, which are only partly alleviated by antiemetics, and also numbness, tinnitus, and reduced hearing. A major problem is an inherent and the development of an acquired platinum resistance of refractory tumors. Various X-ray structure determinations have been performed on cisplatin and its solvent adducts (no water adduct).

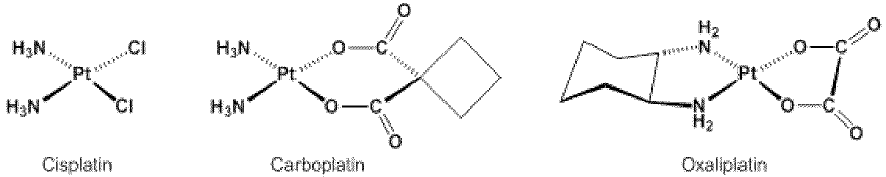

### Structures of Cisplatin, Carboplatin and Oxaliplatin.

Already from early screenings, carboplatin emerged as a further platinum based and clinically applied second generation anti-cancer agent. In carboplatin the rather labile chloride anions of cisplatin are replaced with 1,1-cyclobutanedicarboxylate (CBDCA), representing a dianionic substituted malonate ligand. Carboplatin exhibits lower reactivity and slower DNA binding kinetics, but otherwise appears to act with cell DNA in quite a similar way as cisplatin.

Carboplatin has a very similar activity profile against cancers as for cisplatin. While there are generally less adverse effects, the main drawbacks of carboplatin are that it does not sort out the problem of platinum resistance and its myelosuppressive effect. This causes the blood cell and platelet output of bone marrow to decrease quite dramatically, sometimes to as low as 10% of its usual production levels. Carboplatin is generally less active than cisplatin. This is clinically usually overcome by increasing the dosage to a 4:1 ratio compared to cisplatin. Due to its relative inertness, most of the carboplatin remains unaltered in the blood stream and becomes egested by urine. The structure of carboplatin is known; in addition numerous further Pt-CBDCA complexes and various other Pt-malonate complexes have been structurally investigated.

Oxaliplatin, trans-(*R*,*R*)-1,2-diamino-cyclohexane platinum(II)oxalate, is considered as a third generation Pt-based anti-cancer drug. Instead of NH₃, oxaliplatin contains the chiral trans-1,2-diaminocyclohexane (DACH) ligand as a neutral "carrier ligand". While the also potent Pt₋dichloride (DACH)PtCl₂ is scarcely soluble in water, the oxalate (DACH)Pt(C₂O₄) dissolves well. Oxaliplatin has gained almost worldwide approval in the treatment of colon cancers starting from 1998. Dose-limiting toxicity is neurotoxicity. Interestingly, the trans-(*R*,*R*) enantiomer is more potent than the trans-(*S*,*S*) enantiomer and both of these are more efficient than the cis-(*R*,*S*) isomer. These facts are attributed both to the relative flat structures of the trans isomers, allowing for an easier approach of the trans isomeric complexes to cell DNA, and to the specific hydrogen bonding between the primary amine N-H functions and DNA purine bases. As a consequence, recognition and repair of DNA damage by platinum are impeded, resulting in reduced platinum resistance as compared to the first and second generation drugs.

While cisplatin is administered intravenously due to its high hydrophilicity, an increased lipophilicity of a derivative drug would be required for oral administration and intestinal uptake.

Cisplatin and its homologues obey the general formula cis-Pt(II)A₂Y₂, in which A represents a neutral amine ligand bearing 1-3 protons, and Y (Y₂, respectively) represents an anionic leaving group such as halide, oxalate, and malonate. The amine is viewed as a "carrier ligand" which assists penetration of the neutral drug through the cell membrane. Y undergoes hydrolysis under physiological conditions, fast for chloride and slower for oxalate and malonate.

It emerged from structure-activity relationship studies that certain requirements must be fulfilled by a Pt complex to expose anti-tumor activity. These are, inter alia, two amino functions in cis position at a Pt(II) or Pt(IV) center, with the amino groups possessing H atoms to allow for hydrogen bonding, presumably to the purine bases of the cell DNA. The anionic ligands must be able to slowly hydrolyze under physiologic conditions.

Relatively few Pt(II) complexes with secondary amine ligands have been investigated; in that respect, monodentate amine and chelating acyclic or cyclic diamine ligands can be differentiated.

The inventors of the present invention found that the Pt-bispidine analogs of cisplatin, carboplatin and oxaliplatin show a particular cytotoxicity. The Pt-bispidine complexes of the present invention are apparently the first complexes containing a bicyclic diamine ligand.

It was further found that cytotoxicity, hydro- and lipophilicity and thereby membrane permeability can be adjusted by modification of the bispidine part of the compounds.

In a first aspect, the present invention is directed to a diazabicyclohydrocarbon-Pt complex of the general formula (I):

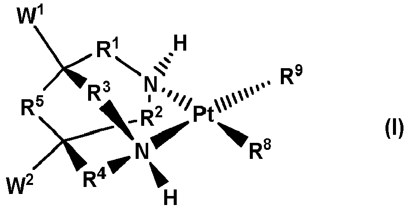

Wherein:
- R¹, R², R³ and R⁴ may be the same or different and may each be C₁-C₃-alkylene, optionally being substituted by one or more substituents selected from the group consisting of -OH, -X, methyl, ethyl, methoxy and ethoxy;
- R⁵ may be -O-, -CO-, -C(OR⁶)(OR⁷)-, -N(R⁶)-, or C₁-C₃-alkylene, said C₁-C₃-alkylene optionally being substituted by one or more substituents selected from the group consisting of -OH, -OR⁶, -NR⁶R⁷, -X, C₁ to C₆ alkyl, C₂ to C₆ alkenyl, C₂ to C₆ alkynyl, C₂ to C₅ heteroaryl, each optionally further substituted by C₁ to C₆ alkyl, -X, C₁ to C₆ halogen substituted alkyl, -OR⁶, - NR⁶R⁷, and -(C=O)R⁶, wherein each of said R⁶ and R⁷ may be the same or different and may be independently selected from the group consisting of hydrogen and C₁ to C₆ alkyl optionally substituted by -OH, -OCH₃, -OC₂H₅, - X, -CH₃, -CH₂X, -CHX₂, and -CX₃, or in the case of -C(OR⁶)(OR⁷)- may together form a C₂ to C₆ aliphatic hydrocarbon ring;
- W¹ and W² may be the same or different and may each be hydrogen, -OH, -X, methyl, methoxy, -CO₂R⁶- wherein R⁶ may have the meaning as given before, and
- R⁷ and R⁹ each represent a monodentate ligand or R⁷ and R⁹ may together form a bidentate ligand, and
- X is halogen.

In the formulae of the inventive compounds, X stands for halogen, in particular F or Cl. In the case of --C(OR⁶)(OR⁷)--, R⁶ and R⁷ may together form a C₂ to C₆ aliphatic hydrocarbon ketal ring which may be prepared by reacting the respective ketone with an α,ω-diol. In some particular embodiments of the complexes of the invention, at least one of R¹ to R⁵ is not methylene. According to the invention, alkylene is to be understood as -(CH₂)- chain with the indicated number of C-atoms. In some embodiments, R⁵ may represent a C₁-C₃-alkylene group wherein said C₁ to C₆ alkyl, C₂ to C₆ alkenyl, or C₂ to C₆ alkynyl substituents may form a hydrocarbon ring with said C₁-C₃-alkylene group, said hydrocarbon ring optionally substituted by -OH, -OCH₃, -OC₂H₅, -X, -CH₃, -CH₂X, -CHX₂, and -CX₃.

In some embodiments, R¹, R², R³ and R⁴ may be the same or different and may also each be C₁-C₃-alkylene being substituted by one or more substituents selected from the group consisting of -OH, -X, C1 to C4 alkyl and C1 to C4 alkoxy.

A preferred embodiment of the present invention encompasses said diazabicyclo-Pt complex of the general formula (I), wherein the C₁-C₃-alkylene group for R⁵ is substituted by one or more substituents selected from the group consisting of - OH, -OR⁶, -NR⁶R⁷, -X, C₁ to C₆ alkyl, C₂ to C₆ alkenyl, C₂ to C₆ alkynyl, C₂ to C₅ heteroaryl, each optionally further substituted by C₁ to C₆ alkyl, -X, C₁ to C₆ halogen substituted alkyl, -OR⁶, -NR⁶R⁷, and -(C=O)R⁶, wherein each of said R⁶ and R⁷ may be the same or different and may be selected from the group consisting of hydrogen or C₁ to C₆ alkyl optionally substituted by -OH, -OCH₃, - OC₂H₅₃ -X, -CH₃, -CH₂X, -CHX₂, and -CX₃, or in the case of -C(OR⁶)(OR⁷)- may together form a C₂ to C₆ aliphatic hydrocarbon ring; and the other substituents are as defined before.

In another embodiment of the present invention of the diazabicyclo-Pt complex of the general formula (I), R⁵ may be -O-, -CO-, -C(OR⁶)(OR⁷)-, -N(R⁶)-, wherein R⁶, R⁷ and the other substituents are as defined before.

Smaller substituents of R¹ to R⁴ that allow for low steric hindrance between the Pt-complex and DNA purine bases are preferred. However, R⁵ can also be substituted by sterically more demanding substituents, as being located on the opposite side of the Pt central atom.

In another embodiment, a diazabicyclo-Pt complex, wherein R¹, R², R³ and R⁴ each represent a methylene or ethylene bridge, each optionally being substituted by one or more substituents selected from the group consisting of hydrogen, -OH, -X (halogen), methyl, ethyl, methoxy and ethoxy, and R⁵ represents -CO-, -C(OR⁶)(OR⁷)-, -N(R⁶)-, wherein R⁶, R⁷ and the other substituents are as defined before, is encompassed.

In case of monodentate ligands, R⁷ and R⁹ can be anionic or neutral and are preferably selected from the group consisting of -X, OH₂, -OCO₂H, -OSO₃H, - OPO₃H₂, acetate and other monoanionic carboxylates, -OR^{a}, -NR^{a}R^{a}, and R^{a}R^{a}NC(O)H, wherein R^{a} may be the same or different and may be independently selected from the group consisting of -H, -CH₃, and -C₂H₅. Preferred examples for the monodentate ligands are -X, -OCO₂H, -OSO₃H, -OPO₃H₂, acetate, water, and -OH. In case that R⁷ and R⁹ are neutral, a corresponding complex anion may be selected from -X, -OCO₂H, -OSO₃H, -OPO₃H₂, acetate.

In case of a bidentate ligand, this ligand may be a bidentate ligand of the formula - (O,S,NH)-R-(NH,S,O)-, wherein R is a bridging C₁ to C₆ hydrocarbon group being optionally substituted by one or more substituents selected from the group consisting of hydrogen, -OH, -OCH₃, -OC₂H₅, -X, -CH₃, -CH₂X, -CHX₂, and -CX₃, straight chain, branched or cyclic C₁ to C₆ alkyl, C₂ to C₆ alkenyl, C₂ to C₆ alkynyl, C₂ to C₅ heteroaryl, halogen, -OR⁶, -NR⁶R⁷, and -(C=O)R⁶, wherein each of said R⁶ and R⁷ may be the same or different and may be selected from the group consisting of hydrogen or C₁ to C₆ alkyl optionally substituted by -OH, -OCH₃, - OC₂H₅₃ -X, -CH₃, -CH₂X, -CHX₂, and -CX₃.

In such case, R of the bidentate ligand of the formula -(O,S,NH)-R-(NH,S,O)- can particularly be a bridging C₁-C₄-alkylene group, being optionally substituted by one or more substituents selected from the group consisting of -OH, -OCH₃, -OC₂H₅, - X, -CH₃, -CH₂X, -CHX₂, and -CX₃, straight chain, branched or cyclic C₁ to C₆ alkyl, C₂ to C₆ alkenyl, C₂ to C₆ alkynyl, C₂ to C₅ heteroaryl, halogen, -OR⁶, -NR⁶R⁷, and -(C=O)R⁶, wherein each of said R⁶ and R⁷ may be the same or different and may be selected from the group consisting of hydrogen or C₁ to C₆ alkyl optionally substituted by -OH, -OCH₃, -OC₂H₅, -X, -CH₃, -CH₂X, -CHX₂, and -CX₃.

In a further embodiment, the bidentate ligand is a bidentate ligand of the formula (O,S,NH)-R-(NH,S,O)- wherein R is a bridging malonate-type ligand such as 1,1-cyclobutanedicarboxylato or a 2,4-pentanedionate ligand, or a -(C=O)-(C=O)-(oxalate) ligand, or an inorganic dianionic ligand such as -OSO₃- and -OPO(OH)O-.

A bidentate ligand is particularly a 1,1-cyclobutanedicarboxylato ligand or a -O-(C=O)-(C=O)-O- ligand.

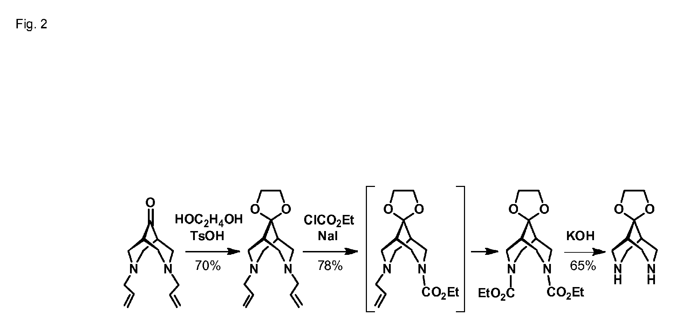

A method for producing the Pt-complexes with the bispidine or bispidine derivatives as defined by general formula (I) is shown in the Reaction Schemes as shown in the Figures 1 to 4.

In more detail, Figures 1 to 4 show:
Fig. 1: General Reaction Scheme for preparing diazabicyclohydrocarbon-Pt complexes of the general formula (I);
Fig. 2: Synthesis of spiro[bispidin-9,2'-[1,3]dioxolane] ((C₂H₄O₂)C₇H₁₀(NH)₂);
Fig. 3: Synthesis of Pt(II)-Bispidin complexes, and
Fig. 4: Synthesis of Pt(II)-Bispidin-9,9-diole complexes.

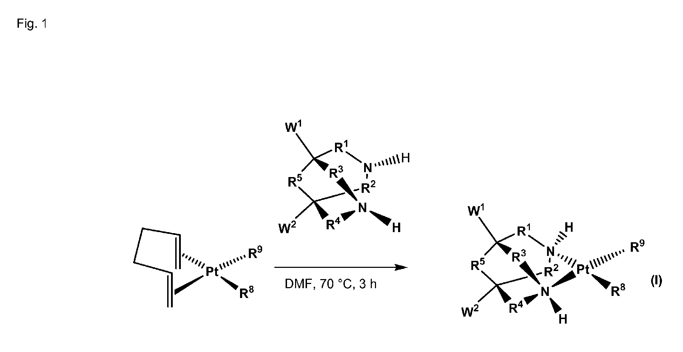

It was found that Pt being coordinated by bispidine or bispidine derivatives as defined by general formula (I) can be produced by reacting the respective diazabicyclo compound with a Pt complex being coordinated by 1,5-hexadiene or derivatives thereof as exemplified in the Reaction Scheme 1 shown in Fig. 1.

Such diazabicyclo-Pt complexes produced by this or any appropriate method can be used as a cytotoxic agent. The inventors of the present invention have carried out pharmaceutical tests for evaluating the pharmaceutical efficacy of the inventive compounds. The results can be taken form the tests below following the preparation of the inventive compounds.

The invention is further illustrated by the following preparation examples.

### Example 1:

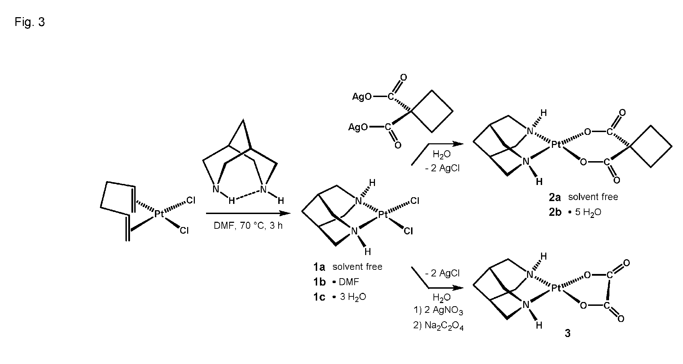

For synthesis of the Pt-bispidine analog of cisplatin, (1,5-hexadiene)PtCl₂ was reacted with the parent bispidine (C₇H₁₄N₂) in DMF at 70 °C for 3 h. By displacement of the 1,5-hexadiene ligand an intense yellow reaction solution is formed. Slow recooling of the solution to ambient temperature afforded large pale yellow crystals of the DMF adduct (C₇H₁₄N₂)PtCl₂·DMF (1b) in 87% yield (Figure 3). No byproducts have been detected in the mother liquor (NMR). An X-ray structure determination revealed a dinuclear structure of 1b in which the DMF ligands are bound by N-H···OCHNMe₂ hydrogen bridge bonds. These appear to be weak, as DMF (b.p. 153 °C) can be fully removed in a vacuum at 50 °C within 24 hours to give solvent free (C₇H₁₄N₂)PtCl₂ (1 a). The latter recrystallizes from the less basic N-methyl formamide (NMF) without inclusion of solvent. Considering that cisplatin does form an adduct with NMF, the NH hydrogen atoms of the bispidine ligand in 1a are apparently less protic than those of the ammonia ligands in cisplatin. No direct synthesis of 1a in NMF was possible due to substantial decomposition of the reaction mixture under these conditions.

While complexes 1a,b appear insoluble in most solvents at ambient temperature, including CH₂Cl₂ and THF, they dissolve increasingly well in water, DMF, and DMSO, in particular when warmed (50 °C). From warm water (C₇H₁₄N₂)PtCl₂·3H₂O (1c) crystallizes at ambient temperature, with only little hydrolysis of the Pt-Cl bonds (NMR).

### Example 2

Synthesis of the Pt-bispidine analog (C₇H₁₄N₂)Pt{(O₂C)₂C₄H₆} (2a) of carboplatin was done by stirring a suspension of complex 1 and the equimolar amount of Ag₂(cbdca) in water in the dark at ambient temperature for 2 days (Figure 3). We found no need to first convert 1 into the iodide, which was suggested by others. The malonate product complex is more soluble in water than the dichloride 1 and remains in solution when AgCl precipitates and is removed by filtration. Rapid evaporation of the water under vacuum by heating the vessel up to 50 °C leaves quantitatively solute-free and pure 2a. Recrystallization of the compound from a smaller amount of water affords the pentahydrate (C₇H₁₄N₂)Pt{(O₂C)₂C₄H₆}·5xH₂O (2b). 2b was characterized by an X-ray structure determination revealing an embedding of the complex molecules in an intricate network of water molecules. Solubility of 2 in DMF is poor.

### Example 3.

Similarly, for the synthesis of the Pt-bispidine analog of oxaliplatin, complex 1 was reacted with 2 equiv of AgNO₃ in aqueous solution to give, besides precipitated AgCl, the soluble diaqua complex [(C₇H₁₄N₂)Pt(OH₂)₂](NO₃)₂. Treatment of the latter with disodium oxalate gave poorly soluble (C₇H₁₄N₂)Pt(C₂O₄) (3) as a colorless precipitate (Figure 3). Recrystallization of 3 from hot water afforded thin colorless needles of 3 in moderate yield (44%). Thus, 3 does not form a water solute compound, unlike 1c and 2b. It furthermore cannot be recrystallized from DMF in which it is hardly soluble up to 100 °C.

### Example 4 - (Spiro[bispidin-9,2'-[1,3]dioxolane])platinum(II)dichloride (4)

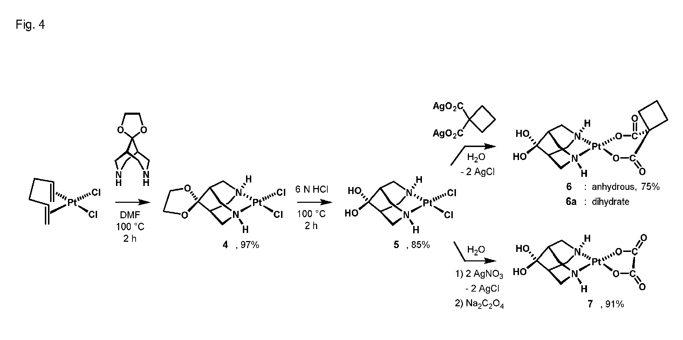

A solution of (C₆H₁₀)PtCl₂ (3.48 g, 10.0 mmol) and spiro[bispidin-9,2'-[1,3]dioxolane] ((C₂H₄O₂)C₇H₁₀(NH)₂, FW184.2) (1.84 g, 10.0 mmol) in 150 mL of DMF was heated to 100 °C for two hours. A light yellow precipitate was formed, which after cooling to ambient temperature was isolated by filtration, washed with diethyl ether, and dried under vacuum: yield 4.36 g (97%) (Figure 4). Anal. Calcd for C₉H₁₆Cl₂N₂O₂Pt (450.2): C, 24.01; H, 3.58; Cl, 15.75; N, 6.22; O, 7.11; Pt, 43.33. Found: Cl, 15.45; N, 6.02; Pt, 42.24. IR (neat): v = 3173 (NH) cm⁻¹. El MS (350 °C): *m*/*e* (%) = 449 ([M]⁺, 3), 413 ([M - HCl]⁺, 3), 377 ([M - 2HCl]⁺ 2), 99 (100). It decomposes above 300 °C without melting. The compound is insoluble in all solvents, including DMSO. No NMR characterization was performed.

### Example 5 - (Bispidin-9,9-diol)platinum(II)dichloride (5)

A suspension of light yellow 4 (450 mg, 1.00 mmol) in 40 mL of 6 N HCl was heated to 100 °C for 2 hours to give a yellow solution. All volatiles were removed in a vacuum to leave a bright yellow residue. This was repeatedly washed with small volumes of cold H₂O and cold ethanol to remove any acid and glycol, and the solid was dried again under vacuum (Figure 4). Recrystallization from boiling water and slow cooling afforded yellow needles: yield 360 mg (85%); mp 320 °C dec. IR (neat): v = 3394 (OH), 3310 (OH), 3223 (NH), 3180 (NH) cm⁻¹. ESlpos MS (DMF): *m*/*e* (%) = 871 ([2M + Na]⁺, 10), 446 ([M + Na]⁺, 100). Anal. Calcd for C₇H₁₄Cl₂N₂O₂Pt (424.2): C, 19.82; H, 3.33; N, 6.60; Cl, 16.72; Pt, 45.99. Found: C, 20.02; H, 3.24; N, 6.71; Cl, 16.78; Pt, 46.28.

### Example 6 - (Bispidin-9,9-diol)platinum(II)(1,1-cyclobutanedicarboxylate) (6)

A warm (50 °C) solution of 5 (424 mg, 1.00 mmol) in water (40 mL) was added to Ag₂(cbdca) (358 mg, 1.00 mmol) suspended in water (10 mL). The reaction mixture was stirred in the dark for 24 hours and the precipitated AgCl was removed by filtration to leave a colorless solution. Concentration of the solution to 20 mL and standing overnight at room temperature gave a first crop of colorless needles. Further concentration of the mother liquor to half of the volume and cooling to 4 °C overnight gave a second crop of the product. According to X-ray diffraction the needles represent the dihydrate 6a. Drying the compound under vacuum at 50 °C overnight afforded the anhydrous 6: yield 370 mg (75%) (Figure 4). The anhydrous 6 decomposes at 260 °C to give black solid. IR (neat): v = 3394 (br, OH), 3161 (br, NH) cm⁻¹. Anal. Calcd for C₁₃H₂₀N₂O₆Pt (495.4): C, 31.52; H, 4.07; N, 5.62; Pt, 39.23. Found: C, 31.38; H, 4.07; N, 5.62; Pt, 39.23. ESlpos MS: m/e (%) = 518 ([M + Na]⁺, 100), 540 ([M + 2 Na - H]⁺, 45). The complex dissolves well in water, DMF, and DMSO.

### Example 7 - (Bispidin-9,9-diol)platinum(II)(oxalate) (7)

Combining a solution of 5 (424 mg, 1.00 mmol) in warm water (40 mL; 50 °C) with a solution of AgNO₃ (325 mg, 1.91 mmol) in water (10 mL) immediately gave a colorless precipitate of AgCl. The mixture was stirred for five hours in the dark. The precipitate was filtered off and to the filtrate was added Na₂C₂O₄ (128 mg, 0.95 mmol). The reaction mixture was stirred overnight and the product was obtained as a colorless precipitate, which was collected by filtration (Figure 4). Recrystallization from hot water afforded thin colorless needles: yield 400 mg (91%); mp 310 °C dec. IR (neat): v = ≈3300 (vbr, OH), 3211 (NH), 3104 (NH) cm⁻¹. Anal. Calcd for C₉H₁₄N₂O₆Pt (441.3): C, 24.49; H, 3.20; N, 6.35; Pt, 44.21. Found: C, 24.42; H, 3.25; N, 6.27; Pt, 44.04. ESlpos MS (DMF): *m*/*e* (%) = 905 ([2M + Na]⁺, 20), 464 ([M + Na]⁺, 100). The compound dissolves only poorly in water and even less so in DMF.

The compounds prepared in Examples 1 to 3 have been further tested for their pharmaceutical efficacy. The biological data have been obtained according to the following MTT cell viability assays.

### MTT cell viability assays.

The rate of cell-survival under the action of test compounds was evaluated by an improved MTT assay as previously described for 3-(4,5-dimethylthiazol-2-yl)-2,5-diphenyltetrazolium bromide (MTT, Applichem, Germany) (Mueller, H., Kassack, M. U., Wiese, M. *J. Biomol. Screen.* 2004, *9*, 506-515).

The assay is based on the ability of viable cells to metabolize yellow MTT to violet formazane crystals that can be detected spectrophotometrically. In brief, A2780 (ECACC, Salisbury, UK) and A2780 CisR cells (established by exposure of A2780 cells to weekly cycles of 2 µmol/L cisplatin over a period of 24 weeks) were seeded at a density of 8,000 cells/well in 96well plates (Corning, Germany) in 90 µL drug-free complete medium. After an incubation period of 24 h at 37 °C under humidified air supplemented with 5% CO₂, cells were exposed to dilutions of the test compounds in 100 µL/well. Stock solutions were prepared in a concentration of 10⁻² M in water, and serial dilutions were prepared by using sodium chloride 0.9%. Incubation was ended after 72 h and cell survival was determined by addition of 25 µL/well MTT solution (5 mg/mL in phosphate buffered saline). After an incubation period of 10 min at 37 °C, the medium was carefully removed. The intracellular formazan product was solubilized by adding 75 µl DMSO/well. Absorbance was measured at 544 nm and 690 nm with a FLUOstar microplate-reader (BMG LabTech, Offenburg, Germany). The absorbance of untreated control cells was taken as 100% viability. All tests were performed in triplicate.

### Data Analysis.

Indicated are mean values ± standard error of the mean. Concentration/inhibition curves were fitted to the data by nonlinear regression analysis using the four parameter logistic equation (GraphPad Prism).

Resistance factor of bispidine analogs 1 a, 2a, 3 and cisplatin, carboplatin, and oxaliplatin in the cisplatin resistance model A2780 / A2780 CisR.

| **Cpd.** | **IC₅₀ [µM]** | | |
|---|---|---|---|
| | **A2780** | **A2780 CisR** | **resistance factor** |
| cisplatin | 1.38 | 16.2 | 11.7 |
| **1a** | 4.20 | 10.6 | 2.52 |
| carboplatin | 4.23 | 40.2 | 9.50 |
| **2a** | 8.84 | 57.1 | 6.46 |
| oxaliplatin | 0.21 | 0.95 | 4.52 |
| **3** | 7.24 | 35.3 | 4.88 |

Values are means ± SEM derived from a representative experiment performed in triplicate. The resistance factor was calculated as the ratio of the IC₅₀ value of the A2780 CisR cells and the IC₅₀ value of the corresponding sensitive parental A2780 cell line.

## Claims

1. A diazabicyclohydrocarbon-Pt complex of the general formula (I): 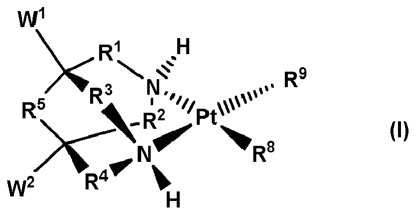 Wherein:
- R¹, R², R³ and R⁴ may be the same or different and may each be C₁-C₃-alkylene, optionally being substituted by one or more substituents selected from the group consisting of -OH, -X, methyl, ethyl, methoxy and ethoxy;
- R⁵ may be -O-, -CO-, -C(OR⁶)(OR⁷)-, -N(R⁶)-, or C₁-C₃-alkylene, said C₁-C₃-alkylene optionally being substituted by one or more substituents selected from the group consisting of -OH, -OR⁶, -NR⁶R⁷, -X, C₁ to C₆ alkyl, C₂ to C₆ alkenyl, C₂ to C₆ alkynyl, C₂ to C₅ heteroaryl, each optionally further substituted by C₁ to C₆ alkyl, -X, C₁ to C₆ halogen substituted alkyl, -OR⁶, -NR⁶R⁷, and -(C=O)R⁶, wherein each of said R⁶ and R⁷ may be the same or different and may be independently selected from the group consisting of hydrogen and C₁ to C₆ alkyl optionally substituted by -OH, -OCH₃, -OC₂H₅, -X, -CH₃, -CH₂X, -CHX₂, and -CX₃, or, in the case of -C(OR⁶)(OR⁷)-, may together form a C₂ to C₆ aliphatic hydrocarbon ring;
- W¹ and W² may be the same or different and may each be hydrogen, -OH, -X, methyl, methoxy, -CO₂R⁶- wherein R⁶ may have the meaning as given before, and
- R⁸ and R⁹ each represent a monodentate ligand or R⁸ and R⁹ may together form a bidentate ligand, and
- X is halogen.

2. Diazabicyclohydrocarbon-Pt complex according to claim 1, wherein R⁵ is a C₁-C₃ alkylene group being optionally substituted by one or more substituents selected from the group consisting of -OH, -OR⁶, -NR⁶R⁷, -X, C₁ to C₆ alkyl, C₂ to C₆ alkenyl, C₂ to C₆ alkynyl, C₂ to C₅ heteroaryl, each optionally further substituted by C₁ to C₆ alkyl, -X, C₁ to C₆ halogen substituted alkyl, -OR⁶, -NR⁶R⁷, and -(C=O)R⁶, wherein each of said R⁶ and R⁷ may be the same or different and may be selected from the group consisting of hydrogen or C₁ to C₆ alkyl optionally substituted by -OH, -OCH₃, -OC₂H₅, -X, -CH₃, -CH₂X, -CHX₂, and -CX₃, or in the case of -C(OR⁶)(OR⁷)-may together form a C₂ to C₆ aliphatic hydrocarbon ring; and the other definitions are as given before.

3. Diazabicyclohydrocarbon-Pt complex according to claim 1 wherein R⁵ is -O-, -CO-, -C(OR⁶)(OR⁷)-, -N(R⁶)-, wherein R⁶, R⁷ and the other definitions are as given before.

4. Diazabicyclohydrocarbon-Pt complex according to claim 1 wherein R¹, R², R³ and R⁴ each represent a methylene or ethylene bridge, each optionally being substituted by one or more substituents selected from the group consisting of -OH, -X, methyl, ethyl, methoxy and ethoxy, and R⁵ represents -CO-, -C(OR⁶)(OR⁷)-, -N(R⁶)-, wherein R⁶, R⁷ and the other substituents are as given before.

5. Diazabicyclohydrocarbon-Pt complex according to any of claims 1 to 4, wherein R⁸ and R⁹ may be independently selected from anionic or neutral monodentate ligands, particularly from the group of monodentate ligands consisting of --X, OH₂, -OCO₂H, -OSO₃H, -OPO₃H₂, monoanionic carboxylates such as acetate, -OR^{a}, -NR^{a}R^{a}, and R^{a}R^{a}NC(O)H, wherein R^{a} may be the same or different and may be independently selected from the group consisting of -H, -CH₃, and -C₂H₅, wherein R⁶, R⁷ and the other substituents are as defined before.

6. Diazabicyclohydrocarbon-Pt complex according to any of claims 1 to 4, wherein the bidentate ligand is a bidentate ligand of the formula -(O,S,NH)-R-(NH,S,O)-, wherein R is a bridging C₁ to C₆ hydrocarbon group being optionally substituted by one or more substituents selected from the group consisting of hydrogen, -OH, -OCH₃, -OC₂H₅, -X, -CH₃, -CH₂X, -CHX₂, and - CX₃, straight chain, branched or cyclic C₁ to C₆ alkyl, C₂ to C₆ alkenyl, C₂ to C₆ alkynyl, C₂ to C₅ heteroaryl, halogen, -OR⁶, -NR⁶R⁷, and -(C=O)R⁶, wherein each of said R⁶ and R⁷ may be the same or different and may be selected from the group consisting of hydrogen or C₁ to C₆ alkyl optionally substituted by -OH, -OCH₃, -OC₂H₅, -X, -CH₃, -CH₂X, -CHX₂, and -CX₃.

7. Diazabicyclohydrocarbon-Pt-complex according to claim 6, wherein R of the bidentate ligand of the formula -(O,S,NH)-R-(NH,S,O)- is a bridging C₁-C₄-alkylene group, being optionally substituted by one or more substituents selected from the group consisting of -OH, -OCH₃, -OC₂H₅, -X, -CH₃, -CH₂X, -CHX₂, and -CX₃, straight chain, branched or cyclic C₁ to C₆ alkyl, C₂ to C₆ alkenyl, C₂ to C₆ alkynyl, C₂ to C₅ heteroaryl, halogen, -OR⁶, -NR⁶R⁷, and - (C=O)R⁶, wherein each of said R⁶ and R⁷ may be the same or different and may be selected from the group consisting of hydrogen or C₁ to C₆ alkyl optionally substituted by -OH, -OCH₃, -OC₂H₅, -X, -CH₃, -CH₂X, -CHX₂, and - CX₃.

8. Diazabicyclohydrocarbon-Pt complex according to any of claims 1 to 4, wherein the bidentate ligand is a bidentate ligand of the formula (O,S,NH)-R-(NH,S,O)- wherein R is a bridging malonate-type ligand such as 1,1-cyclobutanedicarboxylato or a 2,4-pentanedionate ligand, or a -(C=O)-(C=O)-(oxalate) ligand, or an inorganic dianionic ligand such as -OSO₃- and - OPO(OH)O-.

9. Diazabicyclohydrocarbon-Pt complex according to claim 8, wherein the bidentate ligand is a 1,1-cyclobutanedicarboxylato ligand or a -O-(C=O)-(C=O)-O- ligand.

10. Process for preparing a diazabicyclohydrocarbon-Pt complex of the general formula (I), **characterized in that** a 1,5-hexadiene-Pt-R⁸R⁹-complex is reacted with a diazabicyclo-compound of the general formula (II): 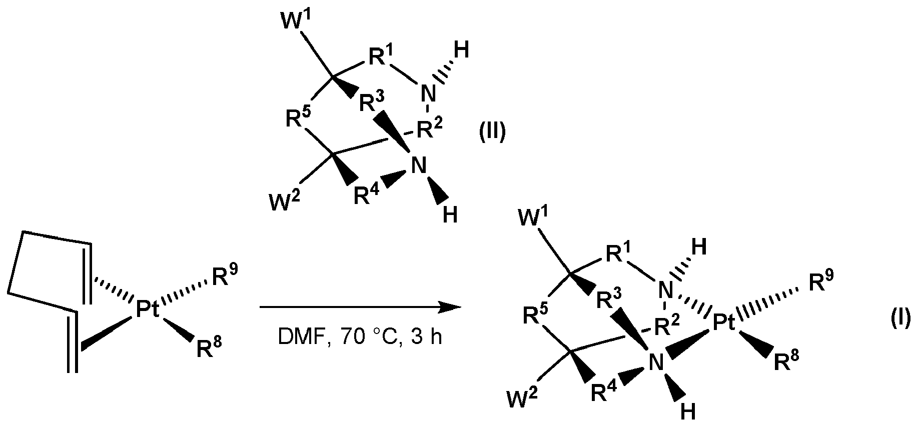 wherein R¹ to R⁵ have the meaning as given in claim 1, and wherein R⁸ and R⁹ each represent a monodentate ligand or R⁸ and R⁹ may together form a bidentate ligand.

11. Use of a diazabicyclohydrocarbon-Pt-complex according to any of claims 1 to 7 as a pharmaceutical, in particular as a cytotoxic agent.
